Europäisches Patentamt

European Patent Office

Office européen des brevets

(19)

(11) Veröffentlichungsnummer: **0 012 156**
**B1**

(12) # EUROPÄISCHE PATENTSCHRIFT

(45) Veröffentlichungstag der Patentschrift:
21.04.82

(51) Int. Cl.³: **A 61 K 9/00**, A 61 K 39/395

(21) Anmeldenummer: **79103152.9**

(22) Anmeldetag: **27.08.79**

(54) **Immunserumglobulin (ISG)-Präparate und Verfahren zu ihrer Herstellung.**

(30) Priorität: **18.12.78 US 970686**

(43) Veröffentlichungstag der Anmeldung:
**25.06.80 Patentblatt 80/13**

(45) Bekanntmachung des Hinweises auf die Patenterteilung.
**21.04.82 Patentblatt 82/16**

(84) Benannte Vertragsstaaten:
**AT BE CH DE FR GB IT SE**

(56) Entgegenhaltungen:
**DE-A-2 653 534**
**GB-A-550 224**
**US-A-3 903 262**
**APPLIED CHEMISTRY AND MICROBIOLOGY,**
**Band 13, Nr. 4, Juli, August 1977,**
**S. E. TUKACHINSKII et al.: »Stabilization of**
**immunoglobulin preparations«, Seiten 473—476**
**CHEMICAL ABSTRACTS, Band 79, Nr. 5,**
**6. August 1973, Zusammenfassung Nr. 28470h,**
**Seite 152, Columbus, Ohio, US**
**S. TEKMAN et al.: »Preventive effect of some**
**sugars and mannitol on the heat coagulation of**
**proteins«**

(73) Patentinhaber: **Cutter Laboratories, Inc., Fourth and
Parker Streets, Berkeley California 94710 (US)**

(72) Erfinder: **Lundblad, John L., 1390 Club View Court, El
Cerrito, CA 94530 (US)**
Erfinder: **Warner, Willis L., 39 Bret Harte Road, San
Rafael, CA 94901 (US)**
Erfinder: **Fernandes, Peter M., 4405 Corkwood Court,
Concord, CA 94521 (US)**

(74) Vertreter: **Dill, Erwin, Dr. et al, c/o BAYER AG
Zentralbereich Patente Marken und Lizenzen Bayerwerk,
D-5090 Leverkusen 1 (DE)**

**CHEMICAL ABSTRACTS, Band 87, Nr. 23, 5. Dezember 1977, Zusammenfassung Nr. 179260f.
Seite 184, Columbus, Ohio, US,
B. A. SCHWARTZ et al.: »Proteins containing
reductively aminated disaccharides. Synthesis
and chemical characterization«**

## Immunserumglobulin-(ISG-)Präparate und Verfahren zu ihrer Herstellung

Die Erfindung betrifft Immunserumglobulin-(ISG-)Präparate, die durch Zusatz von Maltose stabilisiert sind, sowie ein Verfahren zu deren Herstellung.

Es ist bekannt, daß viele Plasmaproteinpräparate, die für die Verabreichung an Menschen oder Tiere vorgesehen sind, Stabilisatoren erfordern, um Denaturierung oder eine sonstige Veränderung vor dem Gebrauch zu verhindern. Instabilität von Proteinpräparaten wird insbesondere in Abhängigkeit von der Konzentration beobachtet. Einige Proteinpräparate, insbesondere Immunserumglobulinpräparate, sind in relativ verdünnten Lösungen (beispielsweise mit einer Konzentration unter 15 Gew.-%) des Proteins besonders instabil. Diese Instabilität, die sich durch Bildung von unlöslichen Teilchen (»Ausflocken« oder »Ausschuppen« = »shedding«) bemerkbar machen kann, nimmt häufig zu, wenn das Proteinpräparat bei Temperaturen oderhalb von Kühlschranktemperatur (etwa Raumtemperatur oder darüber) gelagert wird.

Verschiedene Zusatzstoffe wurden bereits mit unterschiedlichem Grad von Erfolg für die Stabilisierung von Proteinpräparaten verwendet. Beispielsweise wird durch Erhöhen der Konzentration des Proteins oder durch Zusatz eines anderen Proteins, z. B. Albumin, die Stabilität in einigen Fällen gesteigert. Leider sind solche Präparate für therapeutische Zwecke nicht immer unbedenklich. Es ist bekannt, daß Aminosäuren für die Stabilisierung einiger Proteinpräparate brauchbar sind und abgebaute Gelatine gewöhnlich als Stabilisator, insbesondere in europäischen Ländern, verwendet wird. Bei der Erwägung eines geeigneten Stabilisators müssen Faktoren wie mangelnde Antigenwirkung (bei Gelatine möglich), Einfluß auf die Osmolarität der endgültigen Lösung, biologische Aktivität der zu stabilisierenden speziellen Proteine und Verfügbarkeit und Kosten des Stabilisators berücksichtigt werden.

Verschiedene Kohlenhydrate wurden bereits zur Stabilisierung, Erleichterung der Verarbeitung und/oder zur Steigerung der Löslichkeit gewisser biologisch aktiver Proteinpräparate verwendet. Beispielsweise beschreibt die US-PS 2 826 533 die Verwendung von Dextrose zur Steigerung der Löslichkeit eines Fibrinogenpräparats. Die US-PS 4 089 944 beschreibt die Verwendung verschiedener Kohlenhydrate (z. B. Dextrose, Mannose, Galactose, Fructose, Lactose, Saccharose und Maltose) zur Steigerung der Löslichkeit eines AHF-Fibrinogenpräparats. Die Stabilisierung von Plasma mit Invertzucker wird in der US-PS 3 057 781 beschrieben.

Dextrose wird zwar dem Immunserumglobulin (z. B. Intraglobin®, ein modifiziertes Immunserumglobulin) zugesetzt, um die Stabilität und/oder Löslichkeit zu steigern, jedoch hat sich gezeigt, daß die Globuline in handelsüblichen Präparaten mit der Zeit zum Zusammenballen neigen, wodurch die optische Dichte erhöht wird. Dies hat wiederum eine Erscheinung zur Folge, die gewöhnlich als »Ausflocken« (»shedding) bezeichnet wird. Der hier gebrauchte Ausdruck »Ausflocken« bedeutet eine sichtbare Ausfällung von Proteinmolekülen. Es wird angenommen, daß das Ausflocken durch Zusammenballen der Globulinmoleküle verursacht wird, wodurch die Moleküle, insbesondere in verdünnter Lösung, unlöslich werden. Es ist jedoch zu bemerken, daß die genaue Natur des Ausflockens noch nicht völlig aufgeklärt ist. Das Ausflocken ist unerwünscht, da es visuell zu beobachten ist und die Möglichkeit der Deaktivierung oder Denaturierung des ausgeflockten Proteins andeutet und damit die wirksame Menge von verfügbarem Globulin verringert. Darüber hinaus ist eine Globulinlösung, die ausgeflocktes Protein enthält, vom Aussehen her als Produkt unbefriedigend.

Ein weiterer Nachteil, der mit der Verwendung bekannter Zucker als Stabilisatoren in Proteinlösungen verbunden ist, ist die Tatsache, daß gewisse Zuckerlösungen beim Erhitzen zu bräunen pflegen. Außerdem kann es in gewissen Fällen zweckmäßig sein, die Verwendung von schnell assimilierten Zuckern, z. B. Dextrose, in Produkten, die für den menschlichen Gebrauch vorgesehen sind, insbesondere für die Verwendung durch Diabetiker, zu vermeiden.

Überraschenderweise wurde nun ein stabilisiertes Immunserumpräparat entwickelt, das in verdünnter Konzentration über lange Zeiträume im wesentlichen frei von Ausflockungen oder »shedding« ist, und in dem ein gebräuchlicher, verhältnismäßig inerter Zucker verwendet wird, der im allgemeinen in genügenden Mengen verwendet werden kann, um eine pharmazeutisch unbedenkliche isotonische Globulinlösung zu gewährleisten. Dieser Zucker kann verwendet werden, um Immunserumglobulin-(ISG-)Präparate, die sich für die intramuskuläre Verabreichung eignen (IMGG) oder speziell behandelt worden sind, um sie für die intravenöse Verabreichung geeignet zu machen (IVGG), zu stabilisieren. Einzelheiten der stabilisierten Präparate gemäß der Erfindung werden nachstehend erläutert.

Das stabilisierte Immunserumglobulinpräparat gemäß der Erfindung ist eine wäßrige Lösung einer therapeutisch wirksamen Menge von Molekülen von Immunserumglobulin (ISG) und Maltose, wobei die Maltose in einer Menge von 2,5 bis 18 Gewichtsprozent vorliegt, was ein Ausflocken der Globulin-Moleküle bei der Lagerung im wesentlichen verhindert. Gemäß einer bevorzugten Ausführungsform ist das Präparat eine sterile, pharmazeutisch unbedenkliche Lösung von etwa 16,5 Gew.-% IMGG oder 5 bis 10 Gew.-% IVGG und Maltose, wobei die Maltose in einer Menge vorhanden ist, die pharmazeutisch unbedenkliche Isotonie der Globulinlösung sicherstellt. Bei einer anderen bevorzugten Ausführungsform enthält die Globulinlösung 2,5 bis 18 Gew.-% (vorzugsweise 5 bis 15 Gew.-%) Maltose, wobei eine Maltosemenge von etwa 10 Gew.-% besonders bevorzugt wird. Gemäß einer weiteren bevorzugten Ausführungsform enthält die Lösung etwa 10 Gew.-% Maltose und eine gewisse Menge Glycin, vorzugsweise etwa 0,1 Mol Glycin.

Die Erfindung wird nachstehend unter Bezugnahme auf die Abbildungen weiter erläutert.

Fig. 1 ist eine graphische Darstellung, die die Stabilität einer IVGG-Lösung in Abhängigkeit von der Zeit und der Maltosekonzentration bei 57° C veranschaulicht.

Fig. 2 ist eine graphische Darstellung, die die vorteilhaften Wirkungen der Erhöhung der Mengen der Maltose auf die Stabilität einer IVGG-Lösung über einen gewissen Zeitraum bei 57° C veranschaulicht.

Das für die Zwecke der Erfindung verwendete Stabilisierungsmittel ist besonders vorteilhaft für Lösungen von ISG (IMGG oder IVGG) von niedriger Konzentration, bei denen das Ausflocken auf Grund einer verhältnismäßig geringen Proteinmenge wahrscheinlicher und augenfälliger ist.

Das Immunserumglobulin (IMGG) und das für die intravenöse Injektion modifizierte Immunserumglobulin (IVGG) sind bekannt und können nach bekannten Verfahren hergestellt werden. Beispielsweise wird das IMGG (häufig als 16,5gewichtsprozentige Lösung erhältlich) gewöhnlich durch Cohn-Fraktionierung hergestellt (siehe Cohn und Mitarb., J. Am. Chem. Soc. 68 [1946], 459–475 und Oncley u. Mitarb., J. Am. Chem. Soc. 71 [1949], 541–550). Beispiele der Herstellung eines IVGG werden in der US-PS 3 903 262 beschrieben.

Die zur Stabilisierung der wäßrigen Globulinlösungen verwendete Maltose wird ausführlich beispielsweise in The Merck Index, 9. Auflage, Merck & Co., Inc., Rahway, N. J. (1976), beschrieben. Das Disaccharid-Maltose ist in reiner Form leicht erhältlich und hat gute Stabilität in wäßrigen Lösungen bis zu 20 Gew.-%, die ohne Bräunung der Lösung autoklaviert werden können. Physiologisch ist sie in kleinen Mengen praktisch indifferent. Bei intravenöser Verabreichung wird sie durch das spezifische Enzym Maltase, das sich bei den meisten Tierspezies einschließlich des Menschen in vielen Gewebestellen findet, teilweise in Glucose umgewandelt.

Ein Teil der verabreichten Maltose wird durch die menschliche Niere ohne wesentliche Diurese schnell unverändert ausgeschieden. Die Umwandlung in Glucose verläuft allmählich und häufig unnachweisbar, wenn Plasmaglucose in Reihenbestimmungen gemessen wird. Ein Anstieg der zirkulierenden Insulinkonzentrationen ist nicht erkennbar. Über nachteilige Reaktionen wurde selbst nach Verabreichung von bis zu 200 mg Maltose in vier Stunden als 10%ige wäßrige Lösung nicht berichtet. Da Maltose ein Disaccharid ist, ist eine 10%ige Lösung im Menschen ungefähr isotonisch. Der hier gebrauchte Ausdruck »pharmazeutisch annehmbare Isotonie« bezieht sich auf den Bereich der Osmolalität in einer pharmazeutischen Lösung, der im allgemeinen nicht zu wesentlichen lokalen nachteiligen Effekten führt (z. B. Gefäßwandreizung). Zwar variiert die Maltosemenge, die benötigt wird, um eine Osmolalität innerhalb dieses Bereichs zu gewährleisten, in Abhängigkeit von Faktoren wie Aminosäurekonzentration, Salzkonzentration usw., jedoch werden erfindungsgemäß vorzugsweise 5 bis 15 Gew.-% Maltose, insbesondere etwa 10 Gew.-% Maltose in der IMGG- oder IVGG-Lösung verwendet.

Wichtiger bei der Bestimmung der richtigen Maltosemenge ist jedoch die Notwendigkeit, Ausflocken in der IMGG- oder IVGG-Lösung mit der Zeit zu vermeiden. Wie bereits erwähnt, ist »Ausflocken« oder »shedding« ein Ausdruck, der eine wahrnehmbare Ausfällung der Globulinmoleküle bezeichnet, die mit sinkender Konzentration der Proteinlösung besonders stark auftritt. Die Erscheinung des Ausflockens kann genau beobachtet und registriert werden, indem die spektrophotometrische Lichtdurchlässigkeit einer gegebenen Lösung bei 580 nm festgestellt wird. Unterschiede in der Durchlässigkeit mit und ohne variierende Mengen der Maltose stellen ein Mittel zur Bestimmung des Grades des Ausflockens über einen Zeitraum bei einer gegebenen Temperatur dar. Das Ausflocken kann auch visuell festgestellt werden. Im hier gebrauchten Sinne bedeutet eine wesentliche Hemmung des Ausflockens eine Änderung der Durchlässigkeit bei 580 von weniger als 10%, vorzugsweise weniger als 5%, wenn die Lösung 24 Stunden bei 57° C erhitzt wird. Es leuchtet ein, daß das Ausflocken bei niedrigeren Temperaturen langsamer eintritt.

In Voruntersuchungen, die auf das Verständnis der Natur der Erscheinung des Ausflockens gerichtet waren, wurde gefunden, daß eine Änderung des pH-Werts einer 5%igen IVGG-Lösung im endgültigen Gefäß im zulässigen Bereich, d. h. pH 6,4 bis 7,2, keinen Einfluß auf den Grad des Ausflockens hatte. Weitere Untersuchungen ergaben, daß der Zusatz von Nicht-Kohlenhydraten, z. B. NaCl oder Glycin allein, unwirksam für die Stabilisierung der Proteinlösung für mehr als eine sehr kurze Zeit war. Eine Kombination von NaCl und Glycin war ebenfalls unwirksam.

Die vorstehend genannten Untersuchungen ergaben, daß der Test der beschleunigten Ausfällung eine einfache und schnelle Bestimmung der Fähigkeit von IMGG oder IVGG, der Instabilität durch Lagerung unter abträglichen Bedingungen zu widerstehen, sowie einen Hinweis auf das Ausmaß, in dem der Charakter des Moleküls bewahrt worden ist, ermöglicht.

Verschiedene Kohlenhydrate (Dextrose, Fructose, Mannit, Sorbit und Maltose) wurden in verschiedenen Konzentrationen als mögliche Stabilisatoren für die Proteinlösung untersucht. In allen Fällen erwies sich die Stabilität als direkt proportional der Zuckerkonzentration. In allen Fällen war das Material im endgültigen Gefäß bei Verwendung der Kohlenhydrate klarer als Präparate, die Glycin und NaCl enthielten.

Es wurde gefunden, daß Maltose insgesamt der beste Stabilisator hinsichtlich der klinischen Unbedenklichkeit war. Beispielsweise kann Dextrose unerwünschte Hyperglykämie verursachen. Mannit ist bei wirksamen Konzentrationen ein Diuretikum, und Sorbit und Fructose haben nachteilige Wirkungen auf das Säure-Base-Gleichgewicht.

Bei einer Reihe von Versuchen, bei denen die Maltosemenge von 5% auf 18% erhöht wurde, wurde ferner gefunden, daß durch Zusatz von Glycin die Klarheit einer 5%igen IVGG-Lösung bedeutend verbessert wurde. Obwohl somit der Zusatz von Maltose ein stabiles Produkt ergibt, das im wesentlichen frei von Ausflockung ist, wird bei einer besonders bevorzugten Ausführungsform Glycin einbezogen (z. B. etwa 0,1 Mol Glycin bei einer 5%igen IVGG-Lösung). Die Werte, die die Wirkungen von Maltose, Glycin und einer Kombination der beiden auf die Klarheit von IVGG zusammenfassen, sind in der folgenden Tabelle genannt.

Tabelle I

Einfluß von Maltose und Maltose-Glycin auf die Klarheit von IVGG

| Zusatzstoff | | $T_{580}$, % vorher | $T_{580}$, % nachher | Änderung in % |
|---|---|---|---|---|
| 5% Maltose | | 99,05 | 95,69 | 3,39 |
| 7% Maltose | | 99,25 | 96,51 | 2,76 |
| 10% Maltose | | 99,35 | 97,24 | 2,13 |
| 13% Maltose | | 99,25 | 97,87 | 1,39 |
| 18% Maltose | | 99,45 | 98,07 | 1,39 |
| 5% Maltose, | 0,1 Mol Glycin | 99,15 | 97,29 | 1,88 |
| 7% Maltose, | 0,1 Mol Glycin | 99,35 | 97,58 | 1,78 |
| 10% Maltose, | 0,1 Mol Glycin | 99,35 | 98,22 | 1,14 |
| 13% Maltose, | 0,1 Mol Glycin | 99,50 | 98,76 | 0,75 |
| 18% Maltose, | 0,1 Mol Glycin | 99,60 | 98,76 | 0,85 |
| 5% Maltose, | 0,3 Mol Glycin | 99,15 | 98,07 | 1,09 |
| 7% Maltose, | 0,3 Mol Glycin | 99,35 | 98,66 | 0,70 |
| 10% Maltose, | 0,3 Mol Glycin | 99,45 | 98,96 | 0,50 |
| 13% Maltose, | 0,3 Mol Glycin | 99,45 | 99,05 | 0,40 |
| 18% Maltose, | 0,3 Mol Glycin | 99,65 | 99,25 | 0,40 |

Es ist zu bemerken, daß Maltose als Stabilisator besonders bevorzugt wird, da sie ein verhältnismäßig harmloser Zucker ist und genügend Daten über ihre toxikologischen und klinischen Wirkungen vorliegen. Glycin verbessert in Kombination mit Maltose die Klarheit und ist ein allgemein verwendeter Stabilisator für Proteinlösungen. Aus diesem Grunde und auf Grund der Tatsache, daß Glycin auch zur Osmolalität des Produkts beiträgt, wurde gefunden, daß eine bevorzugte Lösung nur 0,1 Mol Glycin und 10% Maltose enthalten sollte. Osmolalitätswerte für einige Maltose-Glycin-Formulierungen, die die verstehenden Feststellungen stützen, sind in der folgenden Tabelle zusammengestellt.

Tabelle II

Einfluß von Zusatzstoffen auf die Osmolalität
einer 5%igen IVGG-Lösung

| Zusatzstoffe plus 5% IVGG | Osmolalität, mOsm/kg |
|---|---|
| 10% Maltose,   0,3 Mol Glycin | 466 |
| 10% Maltose,   0,1 Mol Glycin | 366 |
| 10% Maltose | 300 |
| 0,3 Mol Glycin,   0,45% NaCl | 413 |

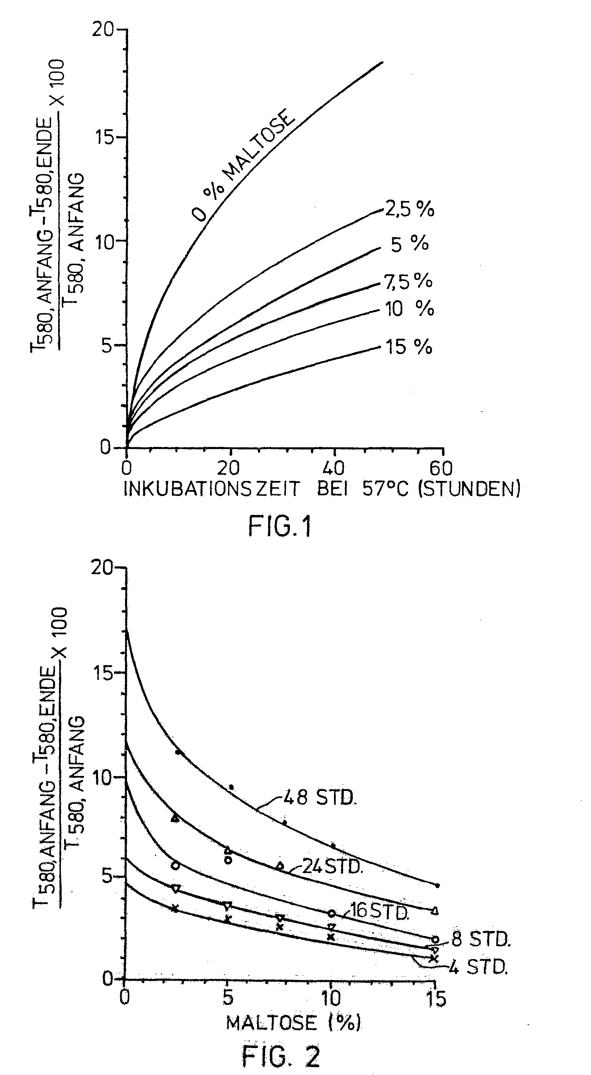

Die vorteilhaften Wirkungen steigender Maltosekonzentrationen auf die Langzeitstabilität (beschleunigte Versuche bei Anwendung einer Temperatur von 57°C) sind in Fig. 1 und Fig. 2 zusammengefaßt, wo die Änderung der Extinktion bei T580 in Abhängigkeit von den Maltosekonzentrationen und von der Zeit bei 57°C eindeutig veranschaulicht wird. Aus Fig. 1 ist ersichtlich, daß das Ausflocken bereits mit 2,4% Maltose praktisch verhindert wurde.

Beispiel

Die Herstellung des Produkts gemäß der Erfindung nach einer besonders bevorzugten Ausführungsform unter Anwendung des besten zur Zeit bekannten Verfahrens wird nachstehend für eine spezielle 5%ige IVGG-Lösung beschrieben. Es ist jedoch zu bemerken, daß der Fachmann auf der Grundlage dieser Beschreibung in der Lage ist, ohne weiteres jede beliebige stabilisierte ISG- oder IVGG-Lösung herzustellen. Das zur Vernaschaulichung beschriebene stabilisierte Produkt gemäß der Erfindung wurde wie folgt hergestellt:

15 g pastenförmige Cohn-Fraktion II werden in 0,45%iger Natriumchloridlösung bei 0 bis 5°C so suspendiert, daß die Proteinkonzentration 5±0,2% beträgt. Die Lösung (etwa 100 ml) wird auf 22 bis 24°C erwärmt und der pH-Wert mit 1 n-Natriumhydroxid auf 8,1±0,1 eingestellt.

Dithiothreitol (Aldrich Chemical Company, Milwaukee, Wisconsin) wird in einer Menge von 0,387 g/l Lösung zugesetzt, worauf man die einsetzende chemische Reduktion 15 Minuten vonstatten gehen läßt. Dann wird Jodacetamid (Aldrich Chemical Company) in einer Konzentration von 1,018 g/l Lösung zugesetzt, worauf man den Alkylierungsprozeß 60 Minuten vonstatten gehen läßt. Der pH-Wert der Lösung wird durch Zusatz von 1 molarem Natriumhydroxid oder 0,8 molarem Natriumacetatpuffer bei 8,1±0,1 gehalten.

Der pH-Wert wird nach Beendigung des Proteinmodifizierungsprozesses auf 6,8 gesenkt und die Lösung zur Entfernung restlicher Reagentien weitgehender Diafiltration unterworfen. Dieser Prozeß besteht in der Diafiltration der Proteinlösung bei wenigstens insgesamt siebenmaligem Volumenersatz gegen die folgenden Lösungen:
0,45%ige Natriumchloridlösungen für die ersten drei Austauschvorgänge;
Wasser für Injektion für wenigstens vier Austauschvorgänge.

Die Lösung wird unter Verwendung eines asbestfreien Filters geklärt, worauf Maltose und Glycin in solchen Mengen zugesetzt werden, daß die endgültige Lösung die folgenden Bestandteile enthält:

| | |
|---|---|
| Protein | 5% |
| Maltose | 10% |
| Glycin | 0,75% (0,1 Mol) |

Der pH-Wert der Lösung wird auf 6,8±0,1 eingestellt und das Material durch Filtration durch ein 0,2-μm-Filter sterilisiert und in geeignete endgültige Gefäße abgefüllt.

**Patentansprüche**

1. Immunserumglobulin-Präparat in Form einer wäßrigen Lösung einer therapeutisch wirksamen Menge von Immunserumglobulin, welche mittels eines Kohlenhydrats stabilisiert ist, dadurch gekennzeichnet, daß die Lösung eine Menge von 2,5 bis 18 Gewichtsprozent Maltose enthält.

2. Immunserumglobulin-Präparat in Form einer sterilen, pharmazeutisch unbedenklichen wäßrigen Lösung für die intravenöse Verabreichung, welches mittels eines Kohlenhydrats stabilisiert ist, dadurch gekennzeichnet, daß es 2,5 bis 18 Gewichtsprozent Maltose enthält.

3. Präparat nach Anspruch 1 und 2, dadurch gekennzeichnet, daß es die Maltose in einer Menge von 5 bis 15 Gewichtsprozent enthält.

4. Präparat nach Anspruch 1 bis 3, dadurch gekennzeichnet, daß die Lösung eine Osmolalität im Bereich von 170 bis 600 mOsm/kg aufweist.

5. Präparat nach Anspruch 1 bis 3, dadurch gekennzeichnet, daß die Lösung eine Osmolalität im Bereich von 260 bis 500 mOsm/kg aufweist.

6. Präparat nach Anspruch 4 oder 5, dadurch gekennzeichnet, daß es als zusätzlichen Stabilisator Glycin enthält.

7. Präparat nach Anspruch 6, dadurch gekennzeichnet, daß die Menge des Glycins etwa 0,1 Mol beträgt.

8. Präparat nach Anspruch 1 und 3 bis 7, dadurch gekennzeichnet, daß das Immunserumglobulin IMGG ist und seine Menge etwa 16,5 Gewichtsprozent beträgt.

9. Präparat nach Anspruch 1 bis 7, dadurch gekennzeichnet, daß das Immunserumglobulin IVGG ist und seine Menge etwa 5 Gewichtsprozent beträgt.

10. Verfahren zur Herstellung eines stabilisierten Immunserumglobulin-Präparats, wobei man eine ISG-Fraktion des Cohn-Prozesses in Natriumchloridlösung suspendiert, den pH-Wert auf 8,1±0,1 einstellt, ein Reduktionsmittel zusetzt, mit Jodacetamid alkyliert, den pH-Wert nach Beendigung des Proteinmodifizierungsprozesses auf 6,8 senkt, die Lösung diafiltriert und klärt und ein Kohlenhydrat als Stabilisator hinzufügt, dadurch gekennzeichnet, daß man der Lösung 2,5 bis 18 Gewichtsprozent Maltose sowie gegebenenfalls Glycin zusetzt.

## Claims

1. An immune serum globulin preparation in the form of an aqueous solution of a therapeutically effective amount of immune serum globulin, which solution is stabilised by means of a carbohydrate, characterised in that the solution contains an amount of 2.5 to 18 percent by weight of maltose.

2. An immune serum globulin preparation in the form of a sterile, pharmaceutically acceptable aqueous solution for intravenous administration, which preparation is stabilised by means of a carbohydrate, characterised in that it contains 2.5 to 18 percent by weight of maltose.

3. The preparation according to Claims 1 and 2, characterised in that it contains an amount of 5 to 15 percent by weight of maltose.

4. The preparation according to Claims 1 to 3, characterised in that the solution has an osmolality in the range from 170 to 600 mOsm/kg.

5. The preparation according to Claims 1 to 3, characterised in that the solution has an osmolality in the range from 260 to 500 mOsm/kg.

6. The preparation according to Claim 4 or 5, characterised in that it contains glycine as an additional stabiliser.

7. The preparation according to Claim 6, characterised in that the amount of glycine is at least about 0.1 mol.

8. The preparation according to Claims 1 and 3 to 7, characterised in that the immune serum globulin is IMGG and the amount of it is about 16.5 percent by weight.

9. The preparation according to Claims 1 to 7, characterised in that the immune serum globulin is IVGG and the amount of it is about 5 percent by weight.

10. Process for the production of a stabilised immune serum globulin preparation, wherein an ISG fraction of the Cohn process is suspended in sodium chloride solution, the pH value is adjusted to 8.1±0.1, a reducing agent is added, alkylation is carried out with iodoacetamide, the pH value is lowered to 6.8 on completion of the protein modification process, the solution is diafiltered and clarified and a carbohydrate is added as stabiliser, characterised in that 2.5 to 18 percent by weight of maltose and optionally glycine are added to the solution.

## Revendications

1. Préparation d'immunoglobuline sérique sous la forme d'une solution aqueuse d'une quantité thérapeutiquement efficace d'une immunoglobuline sérique, qui est stabilisée au moyen d'un hydrate de carbone, caractérisée en ce que la solution contient une quantité de 2,5 à 18% en poids de maltose.

2. Préparation d'immunoglobuline sérique sous la forme d'une solution aqueuse stérile pharmaceutiquement acceptable pour l'administration intraveineuse, qui est stabilisée au moyen d'un hydrate de carbone, caractérisée en ce qu'elle contient 2,5 à 18% en poids de maltose.

3. Préparation suivant les revendications 1 et 2, caractérisée en ce qu'elle contient le maltose en une quantité de 5 à 15% en poids.

4. Préparation suivant les revendications 1 à 3, caractérisée en ce que la solution présente une osmolalité dans la plage de 170 à 600 mOsm/kg.

5. Préparation suivant les revendications 1 à 3, caractérisée en ce que la solution présente une osmolalité dans la plage de 260 à 500 mOsm/kg.

6. Préparation suivant la revendication 4 ou 5, caractérisée en ce qu'elle contient de la glycine comme stabilisant additionnel.

7. Préparation suivant la revendication 6, caractérisée en ce que la quantité de glycine est d'au moins environ 0,1 mole.

8. Préparation suivant les revendications 1 et 3 à 7, caractérisée en ce que l'immunoglobuline sérique est l'IMGG et sa quantité s'élève à environ 16,5% en poids.

9. Préparation suivant les revendications 1 à 7, caractérisée en ce que l'immunoglobuline sérique est l'IVGG et sa quantité s'élève à environ 5% en poids.

10. Procédé de production d'une préparation d'immunoglobuline sérique stabilisée, dans lequel on met en suspension une fraction d'ISG du procédé Cohn dans une solution de chlorure de sodium, on ajuste la valeur du pH à 8,1 ± 0,1, on ajoute un réducteur, on effectue une alkylation à l'iodacétamide, on abaisse le pH à 6,8 lorsque le processus de modification de la protéine est therminé, on effectue une diafiltration et une clarification de la solution et on ajoute un hydrate de carbone somme stabilisant, caractérisé en ce qu'on ajoute à la solution 2,5 à 18% en poids de maltose ainsi que, le cas échéant, de la glycine.

FIG.1

FIG. 2